(19) Europäisches Patentamt
European Patent Office
Office européen des brevets

(11) EP 1 845 085 A1

(12) DEMANDE DE BREVET EUROPEEN

(43) Date de publication:
**17.10.2007 Bulletin 2007/42**

(51) Int Cl.:
***C07D 211/58*** ***(2006.01)*** ***A61K 8/49*** ***(2006.01)***

(21) Numéro de dépôt: **07105821.8**

(22) Date de dépôt: **06.04.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **11.04.2006 FR 0651315**

(71) Demandeur: **L'Oréal**
**75008 Paris (FR)**

(72) Inventeurs:
- **Cavezza, Alexandre**
**93320, PAVILLON SOUS BOIS (FR)**
- **Breton, Philippe**
**78590, NOISY LE ROI (FR)**
- **Dalko, Maria**
**91190, GIF S/YVETTE (FR)**

(74) Mandataire: **Kromer, Christophe**
**L'Oréal, DIPI, River Plaza**
**25-29 Quai Aulagnier**
**92665 Asnieres-sur-Seine (FR)**

# (54) Composition cosmétique antirides

(57) La présente invention concerne l'utilisation cosmétique d'au moins un dérivé de 4-amino pipéridine de formule (I) :

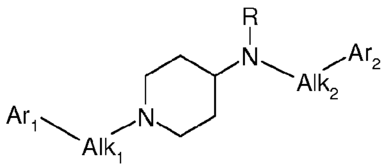

(I) .

dans laquelle :
- $Alk_1$ et $Alk_2$ désignent un radical alkylène en $C_1$-$C_{10}$;
- $Ar_1$ désigne un groupement phényle éventuellement substitué par un ou plusieurs radicaux, identiques ou différents, choisis parmi -F, $-CF_3$, $-R_1$, $-OR_1$, $-NR_1R_2$ ;
- $Ar_2$ désigne un groupement phényle éventuellement substitué par un ou plusieurs radicaux, identiques ou différents, choisis parmi -F, $-CF_3$, $-NR_1R_2$ ;
- R désigne un atome d'hydrogène ou un radical alkyle en $C_1$-$C_{10}$ ;
$R_1$ et $R_2$ désignant un radical alkyle en $C_1$-$C_7$ ;
et leurs sels, isomères optiques et solvates,
comme agent pour lutter contre les rides, notamment les rides d'expression, et/ou décontracter la peau et/ou détendre les traits.

L'invention concerne également une composition cosmétique contenant un tel composé ainsi que les composés nouveaux correspondants.

EP 1 845 085 A1

**Description**

La présente invention concerne l'utilisation de dérivés 4-amino pipéridine comme agent antirides, ainsi qu'une composition cosmétique contenant de tels dérivés destinée à être appliquée sur la peau d'être humains. L'invention concerne aussi de nouveaux dérivés de 4-amino-pipéridine.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en améliorant son renouvellement cellulaire ou encore en favorisant la synthèse, ou en prévenant la dégradation, des fibres élastiques qui composent le tissu cutané.

Bien que ces traitements permettent d'agir sur les rides et ridules dues au vieillissement chronologique ou intrinsèque, ainsi que sur celles dues au photo-vieillissement, ils n'ont pas d'effet sur les rides d'expression, lesquelles nécessitent une intervention sur la composante contractile musculaire (via des agents myorelaxants) ou dermique (via des agents dermo-décontractant) des rides.

Les rides d'expression sont en effet la résultante de mécanismes différents de ceux générant les rides dues au vieillissement.

Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.

Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, le seul moyen couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21).

La Demanderesse a en outre proposé divers composés susceptibles d'offrir un effet anti-rides lorsqu'ils sont appliqués topiquement sur la peau, permettant ainsi d'agir par une autre voie sur les rides d'expression.

Il reste toutefois le besoin de disposer d'autres composés efficaces pour lisser ou estomper les rides, en particulier d'expression.

Or, la Demanderesse a découvert avec étonnement que certains dérivés de 4-amino pipéridine permettaient de satisfaire ce besoin.

La présente invention a donc pour objet l'utilisation cosmétique d'au moins un dérivé de 4-amino pipéridine de formule (I) :

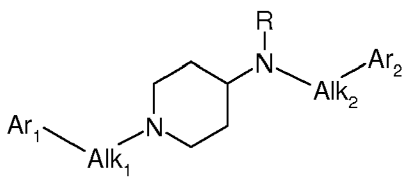

(I) .

dans laquelle :

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;

- $Ar_1$ désigne un groupement phényle éventuellement substitué par un ou plusieurs radicaux, identiques ou différents, choisis parmi -F, $-CF_3$, $-R_1$ , $-OR_1$ , $-NR_1 R_2$ ;

- $Ar_2$ désigne un groupement phényle éventuellement substitué par un ou plusieurs radicaux, identiques ou différents,

choisis parmi -F, $-CF_3$, $-NR_1R_2$ ;

- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi $-Ar_1$, $-OR_1$, $-NR_1R_2$ ;

$R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ;
et leurs sels, isomères optiques et solvates,
comme agent pour lutter contre les rides, notamment les rides d'expression, et/ou décontracter la peau et/ou détendre les traits.

L'invention a aussi pour objet un procédé de traitement cosmétique de la peau, notamment d'une peau ridée, en particulier la peau du visage et/ou du front, comprenant l'application topique sur ladite peau d'une composition comprenant, dans un milieu physiologiquement acceptable, au moins un dérivé de 4-amino pipéridine de formule (I) tel que défini précédemment.

Certains des composés de formule (I) sont connus de l'art antérieur :

- la 1-(2-phényléthyl)-4-(N-benzyl)-amino pipéridine (composé n° 2) est un composé connu ayant la référence CAS 733674-39-0 ;
- la 1-N-benzylamino 4-benzyl pipéridine est notamment décrit comme intermédiaire de synthèse dans la demande WO 2005/005395 (page 61, exemple A-2) ;
- la 1-(2-phényléthyl)-4-[N-(2-phényléthyl)]-amino pipéridine (composé n°4) est décrite dans la demande HU 157777325 ;
- la 1-(2-phényléthyl) 4-[N-(1-phényléthyl)]-amino pipéridine (composé n° 5) est un composé commercialisé par la société Chembridge sous la référence 5454694;
- la 1-benzyl-4-[N-(1-phényléthyl)]-amino pipéridine (composé n° 6) est décrite dans la publication Corruble A. et al, « Structure-selectivity relationship in alkyllithium-aldehyde condensations using 3-aminopyrrolidine lithium amides as chiral auxiliaries » , J. Org. Chem, 1998, 63, 8266-8275 (composé 19b') ;

La demande WO 02/083641 décrit des composés amino-aza-cyclohexanes (en particulier les composés des exemples 10, 11 et 19) dans des compositions pharmaceutiques destinées au traitement de la malaria.

L'invention a encore pour objet une composition comprenant, dans un milieu physiologiquement acceptable, au moins un dérivé de 4-amino pipéridine de formule (II):

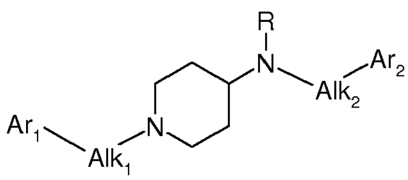

(II)

dans laquelle :

- R désigne un atome d'hydrogène, et
- $Alk_1$ désigne un radical alkylène (radical divalent) linéaire en $C_2$-$C_{10}$ ou ramifié en $C_3$-$C_{10}$ , saturé ou insaturé ; et
- $Alk_2$ désigne un radical alkylène (radical divalent) linéaire saturé en $C_1$-$C_{10}$ ou insaturé en $C_2$-$C_{10}$ ou ramifié en $C_3$-$C_{10}$ saturé ou insaturé ;

ou

- R désigne un radical alkyle linéaire en $C_1$-$C_{10}$, ou ramifié en $C_3$-$C_{10}$, saturé ou insaturé, éventuellement substitué par un groupement choisi parmi $-Ar_1$, $-OR_1$, $- NR_1R_2$ ; et

- $Alk_1$ et $Alk_2$ désignent, indépendamment l'un de l'autre, un radical alkylène (radical divalent) linéaire saturé en $C_1$-$C_{10}$ ou insaturé en $C_2$-$C_{10}$ ou ramifié en $C_3$-$C_{10}$ saturé ou insaturé ;

- $Ar_1$ désigne un groupement phényle éventuellement substitué par un ou plusieurs radicaux, identiques ou différents,

choisis parmi -F, $-CF_3$, $-R_1$ , $-OR_1$ , $-NR_1 R_2$ ;

- $Ar_2$ désigne un groupement phényle éventuellement substitué par un ou plusieurs radicaux, identiques ou différents, choisis parmi -F, $-CF_3$, $-NR_1R_2$ ;

$R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ;
et leurs sels, isomères optiques et solvates.

L'invention a également pour objet de nouveaux composés de 4 amino-pipéridine de formule (III) :

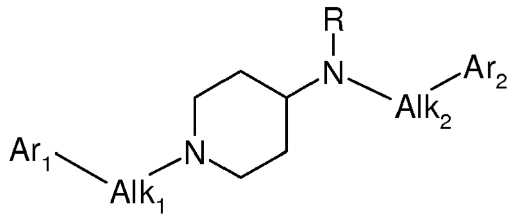

(III)

dans laquelle :

- $Alk_1$ désigne un radical alkylène (radical divalent) linéaire en $C_2$-$C_{10}$ ou ramifié en $C_3$-$C_{10}$, saturé ou insaturé ;
- $Alk_2$ désigne un radical alkylène (radical divalent) linéaire ou ramifié, saturé ou insaturé, en $C_3$-$C_{10}$ ;
- $Ar_1$ désigne un groupement phényle éventuellement substitué par un ou plusieurs radicaux, identiques ou différents, choisis parmi -F, $-CF_3$, $-R_1$, $-OR_1$, $-NR_1R_2$ ;
- $Ar_2$ désigne un groupement phényle éventuellement substitué par un ou plusieurs radicaux, identiques ou différents, choisis parmi -F, $-CF_3$, $-NR_1R_2$ ;
- R désigne un atome d'hydrogène ou un radical alkyle linéaire en $C_1$-$C_{10}$ ou ramifié en $C_3$-$C_{10}$, saturé ou insaturé, éventuellement substitué par un groupement choisi parmi $-Ar_1$, $-OR_1$, $-NR_1R_2$;

$R_1$ et $R_2$ désignant, indépendamment l'un de l'autre, un radical alkyle, saturé ou insaturé, linéaire en $C_1$-$C_7$, ou ramifié ou cyclique en $C_3$-$C_7$ ;
et leurs sels, isomères optiques et solvates.

Dans les formules (I), (II) et (III), les groupes alkyle peuvent notamment être choisis, selon le cas, parmi les groupes : méthyle, éthyle, n-propyle, isopropyle, n-butyle, isobutyle, tert-butyle, pentyle, hexyle, heptyle, octyle, nonyle, décyle. De la même façon, les groupes alkylènes divalents peuvent être choisis parmi les radicaux méthylène, éthylène, n-propylène, isopropylène, n-butylène, isobutylène, tert-butylène, pentylène, hexylène, heptylène, octylène, nonylène, décylène.

Pour les composés de formule (I), on préfère ceux ayant les significations suivantes :

- $Alk_1$ et $Alk_2$ désignent, indépendamment l'un de l'autre, un radical alkylène linéaire en $C_1$-$C_{10}$ ou ramifié en $C_3$-$C_{10}$ saturé ;
- $Ar_1$ désigne un groupement phényle éventuellement substitué par un ou plusieurs radicaux, identiques ou différents, choisis parmi $-R_1$ , $-OR_1$ ;
- $Ar_2$ désigne un groupement phényle éventuellement substitué par un ou plusieurs radicaux $-CF_3$ ;
- R désigne un atome d'hydrogène ou un radical alkyle linéaire en $C_1$-$C_4$, ou ramifié en $C_3$-$C_4$, saturé, éventuellement substitué par un groupement choisi parmi $-Ar_1$, $-OR_1$, - $NR_1R_2$ ;

$R_1$ et $R_2$ désignant, indépendamment l'un de l'autre, un radical alkyle saturé linéaire en $C_1$-$C_4$.

Préférentiellement, on utilise des composés de formule (I) pour lesquels :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi -$Ar_1$, -$OR_1$, - $NR_1R_2$ ;

$R_1$ et $R_2$ désignant, indépendamment l'un de l'autre, un radical alkyle saturé linéaire en $C_1$-$C_4$.

Plus préférentiellement, on utilise des composés de formule (I) pour lesquels :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi un radical phényle, -$NR_1R_2$ , et de préférence un atome d'hydrogène.

Pour les composés de formule (II), on préfère ceux ayant les significations suivantes :

- R désigne un atome d'hydrogène , et
- $Alk_1$ désigne un radical alkylène linéaire en $C_2$-$C_{10}$ ou ramifié en $C_3$-$C_{10}$ , saturé ; et
- $Alk_2$ désigne un radical alkylène linéaire en $C_1$-$C_{10}$ ou ramifié en $C_3$-$C_{10}$ , saturé ;

ou

- R désigne un radical alkyle linéaire en $C_1$-$C_4$, ou ramifié en $C_3$-$C_4$, saturé, éventuellement substitué par un groupement choisi parmi -$Ar_1$, -$OR_1$, -$NR_1$ $R_2$ ; et
- $Alk_1$ et $Alk_2$ désignent, indépendamment l'un de l'autre, un radical alkylène linéaire en $C_1$-$C_{10}$ ou ramifié en $C_3$-$C_{10}$ saturé (notamment tels que ceux décrits précédemment lorsque R = H) ;
- $Ar_1$ désigne un groupement phényle éventuellement substitué par un ou plusieurs radicaux, identiques ou différents, choisis parmi -$R_1$ , -$OR_1$ ;
- $Ar_2$ désigne un groupement phényle éventuellement substitué par un ou plusieurs radicaux -$CF_3$ ;

$R_1$ et $R_2$ désignant, indépendamment l'un de l'autre, un radical alkyle saturé linéaire en $C_1$-$C_4$.

Préférentiellement, on utilise des composés de formule (II) pour lesquels :

- R désigne un atome d'hydrogène , et
- $Alk_1$ désigne un radical alkylène linéaire en $C_2$-$C_4$ ou ramifié en $C_3$-$C_4$, saturé ; et
- $Alk_2$ désigne un radical alkylène linéaire en $C_1$-$C_4$ ou ramifié en $C_3$-$C_4$, saturé ;

ou

- R désigne un radical alkyle linéaire en $C_1$-$C_4$, ou ramifié en $C_3$-$C_4$, saturé, éventuellement substitué par un groupement choisi parmi -$Ar_1$, -$OR_1$, -$NR_1$ $R_2$ ; et
- $Alk_1$ et $Alk_2$ désignent, indépendamment l'un de l'autre, un radical alkylène linéaire en $C_1$-$C_4$ ou ramifié en $C_3$-$C_4$ saturé (notamment tels que ceux décrits précédemment lorsque R = H) ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .

Plus préférentiellement, on utilise des composés de formule (II) pour lesquels :

- R désigne un atome d'hydrogène, et
- $Alk_1$ désigne un radical alkylène linéaire en $C_2$-$C_{10}$ ou ramifié en $C_3$-$C_{10}$ , saturé ; et
- $Alk_2$ désigne un radical alkylène linéaire en $C_1$-$C_{10}$ ou ramifié en $C_3$-$C_{10}$, saturé ;

ou

- R désigne un radical alkyle linéaire en $C_1$-$C_4$, ou ramifié en $C_3$-$C_4$, saturé, éventuellement substitué par un groupement choisi parmi un radical phényle, -$NR_1R_2$, et
- $Alk_1$ et $Alk_2$ désignent, indépendamment l'un de l'autre, un radical alkylène linéaire en $C_1$-$C_4$ ou ramifié en $C_3$-$C_4$ saturé (notamment tels que ceux décrits précédemment lorsque R = H) ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .
De manière plus préférée, on utilise des composés de formule (II) pour lesquels :

- R désigne un atome d'hydrogène,
- $Alk_1$ désigne un radical alkylène linéaire en $C_2$-$C_{10}$ ou ramifié en $C_3$-$C_{10}$ , saturé ;
- $Alk_2$ désigne un radical alkylène linéaire en $C_1$-$C_{10}$ ou ramifié en $C_3$-$C_{10}$ , saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .
Pour les composés de formule (III), on préfère ceux ayant les significations suivantes :

- R désigne un atome d'hydrogène, et
- $Alk_1$ désigne un radical alkylène linéaire en $C_2$-$C_{10}$ ou ramifié en $C_3$-$C_{10}$, saturé ; et
- $Alk_2$ désigne un radical alkylène linéaire ou ramifié en $C_3$-$C_{10}$ , saturé ;

ou

- R désigne un radical alkyle linéaire en $C_1$-$C_4$, ou ramifié en $C_3$-$C_4$, saturé, éventuellement substitué par un groupement choisi parmi -$Ar_1$, -$OR_1$, -$NR_1$ $R_2$ ; et
- $Alk_1$ et $Alk_2$ désignent, indépendamment l'un de l'autre, un radical alkylène linéaire en $C_1$-$C_{10}$ ou ramifié en $C_3$-$C_{10}$ saturé (notamment tels que ceux décrits précédemment lorsque R = H) ;
- $Ar_1$ désigne un groupement phényle éventuellement substitué par un ou plusieurs radicaux, identiques ou différents, choisis parmi -$R_1$ , -$OR_1$ ;
- $Ar_2$ désigne un groupement phényle éventuellement substitué par un ou plusieurs radicaux -$CF_3$ ;

$R_1$ et $R_2$ désignant, indépendamment l'un de l'autre, un radical alkyle saturé linéaire en $C_1$-$C_4$.
Préférentiellement, on utilise des composés de formule (III) pour lesquels :

- R désigne un atome d'hydrogène, et
- $Alk_1$ désigne un radical alkylène linéaire en $C_2$-$C_4$ ou ramifié en $C_3$-$C_4$ , saturé ; et
- $Alk_2$ désigne un radical alkylène linéaire ou ramifié en $C_3$-$C_4$ , saturé ;

ou

- R désigne un radical alkyle linéaire en $C_1$-$C_4$, ou ramifié en $C_3$-$C_4$, saturé, éventuellement substitué par un groupement choisi parmi -$Ar_1$, -$OR_1$, -$NR_1$ $R_2$ ; et
- $Alk_1$ et $Alk_2$ désignent, indépendamment l'un de l'autre, un radical alkylène linéaire en $C_1$-$C_4$ ou ramifié en $C_3$-$C_4$ saturé (notamment tels que ceux décrits précédemment lorsque R = H) ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$.
Plus préférentiellement, on utilise des composés de formule (III) pour lesquels :

- R désigne un atome d'hydrogène, et
- $Alk_1$ désigne un radical alkylène linéaire en $C_2$-$C_4$ ou ramifié en $C_3$-$C_4$ , saturé ; et
- $Alk_2$ désigne un radical alkylène linéaire ou ramifié en $C_3$-$C_4$ , saturé ;

ou

- R désigne un radical alkyle linéaire en $C_1$-$C_4$, ou ramifié en $C_3$-$C_4$, saturé, éventuellement substitué par un groupement choisi parmi un radical phényle, $-NR_1R_2$ , et
- $Alk_1$ et $Alk_2$ désignent, indépendamment l'un de l'autre, un radical alkylène linéaire en $C_1$-$C_4$ ou ramifié en $C_3$-$C_4$ saturé (notamment tels que ceux décrits précédemment lorsque R = H) ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$.
De manière plus préférée, on utilise des composés de formule (III) pour lesquels :

- R désigne un atome d'hydrogène, et
- $Alk_1$ désigne un radical alkylène linéaire en $C_2$-$C_4$ ou ramifié en $C_3$-$C_4$ , saturé ; et
- $Alk_2$ désigne un radical alkylène linéaire ou ramifié en $C_3$-$C_4$ , saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .
Les sels acceptables des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.
Les sels préférés sont ceux obtenus à partir des acides chlorhydrique, sulfurique, acétique, tartrique, citrique.
Les solvates acceptables des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.
Parmi les composés de formule (I) on peut citer :

| Composé | NOM | $Ar_1$ | $Alk_1$ | $Alk_2$ | $Ar_2$ | R |
|---|---|---|---|---|---|---|
| **1** | 1-(2-phényléthyl)-4-[N-(3-phénylpropyl)]-amino pipéridine | Ph | $CH_2CH_2$ | $CH_2CH_2CH_2$ | Ph | H |
| **2** | 1-(2-phényléthyl)-4-(N-benzyl)-amino pipéridine (composé connu) | Ph | $CH_2CH_2$ | $CH_2$ | ph | H |
| **3** | 1-(2-phényléthyl)-4-[N-(3,5-bistrifluorométhyl) benzyl]-amino pipéridine | Ph | $CH_2CH_2$ | $CH_2$ | (3,5)-$CF_3$-Ph | H |
| **4** | 1-(2-phényléthyl)-4-[N-(2-phényléthyl)]-amino pipéridine (composé connu) | Ph | $CH_2CH_2$ | $CH_2CH_2$ | Ph | H |
| **5** | 1-(2-phényléthyl) 4-[N-(1-phényléthyl)]-amino pipéridine (composé connu) | Ph | $CH_2CH_2$ | $CHCH_3$ | Ph | H |
| **6** | 1-benzyl-4-[N-(1-phényléthyl)]-amino pipéridine (composé connu) | Ph | $CH_2$ | $CHCH_3$ | Ph | H |
| **7** | 1-[2-(3,4-diméthoxy)phényléthyl]-4-[N-(3-phénylpropyl)]-amino pipéridine | (3,4)-OMe-Ph | $CH_2CH_2$ | $CH_2CH_2CH_2$ | Ph | H |
| **8** | 1-(3-phénylpropyl)-4-[N-(3-phénylpropyl)]-amino pipéridine | Ph | $CH_2CH_2CH_2$ | $CH_2CH_2CH_2$ | Ph | H |
| **9** | 1-(2-phényléthyl)-4-[N-(4-phénylbutyl)]-amino pipéridine | Ph | $CH_2CH_2$ | $CH_2CH_2CH_2CH_2$ | Ph | H |
| **10** | 1-(2-phényléthyl)-4-[N-(1-phénylpropyl)]-amino pipéridine | Ph | $CH_2CH_2$ | $CHCH_2CH_3$ | Ph | H |
| **11** | 1-[2-(4-tert-butyl)-phényléthyl]-4-[N-(3-phénylpropyl)]-amino pipéridine | 4-tBu-Ph | $CH_2CH_2$ | $CH_2CH_2CH_2$ | Ph | H |
| **12** | 1-(2-phényléthyl)-4-[N-(1S)-1-phényléthyl]-amino pipéridine | Ph | $CH_2CH_2$ | $CHCH_3$(S) | Ph | H |
| **13** | 1-[2-(4-tert-butyl)-phényléthyl]-4-[N-(1-phényléthyl)]-amino pipéridine | 4-tBu-Ph | $CH_2CH_2$ | $CHCH_3$ | Ph | H |
| **14** | 1-(4-phénylbutyl)-4-[N-(3-phénylpropyl)]-amino pipéridine | Ph | $CH_2CH_2CH_2CH_2$ | $CH_2CH_2CH_2$ | Ph | H |
| **15** | 1-(3-phénylpropyl)-4-[N-(1-phénylpropyl)]-amino pipéridine | Ph | $CH_2CH_2CH_2$ | $CHCH_2CH_3$ | Ph | H |
| **16** | 1-(3-phénylpropyl)-4-[N-(1S)-1-phényléthyl]-amino pipéridine | Ph | $CH_2CH_2CH_2$ | $CHCH_3$(S) | Ph | H |
| **17** | 1-(2-phényléthyl)-4-[N-methyl-N-(3-phénylpropyl)]-amino pipéridine | Ph | $CH_2CH_2$ | $CH_2CH_2CH_2$ | Ph | Me |
| **18** | 1-(2-phényléthyl)-4-[N,N-di-(2-phényléthyl)]-amino pipéridine | Ph | $CH_2CH_2$ | $CH_2CH_2$ | Ph | $CH_2CH_2Ph$ |

(suite)

| Composé | NOM | $Ar_1$ | $Alk_1$ | $Alk_2$ | $Ar_2$ | R |
|---|---|---|---|---|---|---|
| **19** | 1-(2-phényléthyl)-4-[N-éthyl-N-(1-phényléthyl)]-amino pipéridine | Ph | $CH_2CH_2$ | $CHCH_3$ | Ph | Et |
| **20** | 1-(2-phényléthyl)-4-[N-(2-phénylpropyl)-N-(1-phényléthyl)]-amino pipéridine | Ph | $CH_2CH_2$ | $CHCH_3$ | Ph | $CH_2CH_2CH_2Ph$ |
| **21** | 1-(2-phényléthyl)-4-[N-(3-phénylpropyl)-N-phényléthyl]amino pipéridine | Ph | $CH_2CH_2$ | $CH_2CH_2CH_2$ | Ph | $CH_2CH_2Ph$ |
| **22** | 1-(2-phényléthyl)-4-[N-benzyl-N-(3-phénylpropyl)]-amino pipéridine | Ph | $CH_2CH_2$ | $CH_2CH_2CH_2$ | Ph | $CH_2Ph$ |
| **23** | 1-(2-phényléthyl)-4-[N-éthyl-N-(3-phénylpropyl)]-amino pipéridine | Ph | $CH_2CH_2$ | $CH_2CH_2CH_2$ | Ph | Et |
| **24** | 1-(2-phényléthyl)-4-[N-propyl-N-(3-phényl propyl)]-amino pipéridine | Ph | $CH_2CH_2$ | $CH_2CH_2CH_2$ | Ph | Pr |
| **25** | 1-(2-phényléthyl)-4-[N-butyl-N-3-(3-phényl propyl)]-amino pipéridine | Ph | $CH_2CH_2$ | $CH_2CH_2CH_2$ | Ph | n-Bu |
| **26** | 1-(2-phényléthyl)-4-[N-2-(3,4-diméthoxy) phényléthyl-N-(3-phénylpropyl)]-amino pipéridine | Ph | $CH_2CH_2$ | $CH_2CH_2CH_2$ | Ph | Ph [(3,4)-OMe-] |
| **27** | 1-(2-phényléthyl)-4-[N-2-diméthylaminoéthyl-N-(3-phénylpropyl)]-amino pipéridine | Ph | $CH_2CH_2$ | $CH_2CH_2CH_2$ | Ph | $(CH_2)_2N(Me)_2$ |
| **28** | 1-(2-phényléthyl)-4-[N-2-méthoxyéthyl-N-(3-phénylpropyl)]-amino pipéridine | Ph | $CH_2CH_2$ | $CH_2CH_2CH_2$ | Ph | $CH_2CH_2OCH_3$ |

Parmi les composés de formule (II) on peut citer ceux précédemment cités à l'exception du composé 6.
Parmi les composés de formule (III) on peut citer les composés 1, 7 à 11 et 14 à 28 précédemment cités.
Les composés (I) particulièrement préférés sont les composés 1, 5, 6, 21, 25, 27, et notamment 1 et 5.
Les composés (II) particulièrement préférés sont les composés 1, 5, 21, 25, 27, et notamment 1 et 5.
Les composés (III) particulièrement préférés sont les composés 1, 21, 25, 27, et notamment 1.
De manière générale, les composés de formule (I) pour lesquels R est un atome d'hydrogène peuvent être préparés selon les schémas I et II ci-après par alkylation du iodure du 1,1-diméthyl 1,1-diméthyl-4-oxopipéridinium avec une alkylamine (III), notamment à une température comprise entre 40 °C et 100 °C, en particulier en présence d'une base minérale (par exemple le bicarbonate de sodium ou la soude) dans un mélange eau/éthanol pour former une pipéridone (A) qui est isolée puis purifiée par exemple par chromatographie sur gel de silice.
La pipéridone (A) obtenue est ensuite alkylée et réduite avec une alkylamine (B) notamment en présence d'acide acétique (1 equivalent molaire) et de $NaBH(OAc)_3$ (triacétoxy borohydrure de sodium) (2 équivalents molaire), en particulier dans le dichlorométhane, et notamment à la température ambiante (25 °C).
Le milieu réactionnel est ensuite extrait 3 fois par de l'eau à pH acide. Les phases aqueuses sont ensuite rassemblées et extraites 3 fois avec du dichlorométhane. Les phases organiques sont rassemblées, séchées et concentrées pour obtenir le produit attendu (Ia), ce dernier pouvant éventuellement être purifié sur gel de silice et/ou par précipitation.

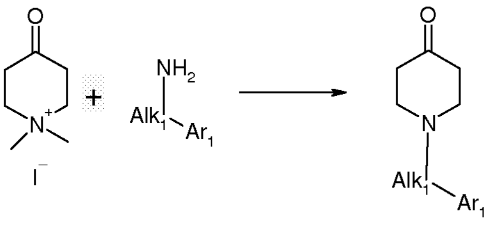

(A)

Schéma I

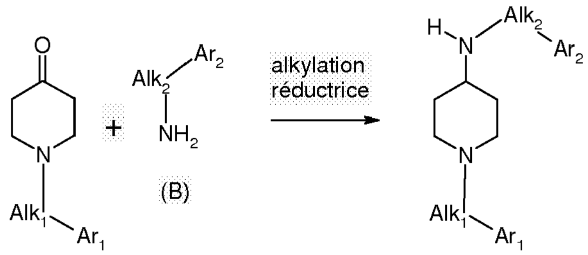


(B)

(A) (Ia)

Schéma II

Les composés 1 à 16 ont été synthétisés selon le procédé général du schéma II.
Les composés de formule (I) pour lesquels R est différent d'un atome d'hydrogène peuvent être préparés selon le schéma III décrit ci-après.
Le composé (Ia) est acylé par le chlorure d'acide de formule RCOCl en présence d'une base organique (par exemple la pyridine ou la triéthylamine) pour conduire à un amide intermédiaire qui est réduit en présence de $LiAlH_4$ (hydrure double de lithium et d'aluminium) notamment dans un solvant éthéré (par exemple le dioxane, le diéthyléther, le tétrahydrofurane), notamment en chauffant à une température comprise entre 40 °C et 100 °C, et permet ainsi d'obtenir le composé (Ib).

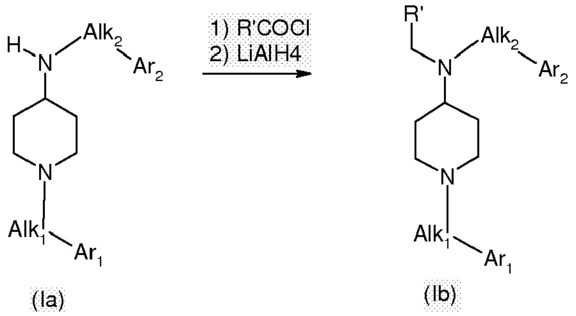


(Ia)

(Ib)

Schéma III

Les composés 17 à 28 ont été synthétisés suivant ce procédé général du schéma III.

La quantité de dérivé de 4-amino pipéridine de formule (I), (II) ou (III) utilisable selon l'invention est bien entendu fonction de l'effet recherché et peut donc varier dans une large mesure.

Pour donner un ordre de grandeur, on peut utiliser les composés de formule (I), (II) ou (III) en une quantité représentant de 0,01% à 10% du poids total de la composition, préférentiellement en une quantité représentant de 0,05% à 5% du poids total de la composition, plus préférentiellement en une quantité représentant de 0,1% à 2% du poids total de la composition.

La composition selon l'invention est adaptée à une application topique sur la peau et elle contient donc un milieu physiologiquement acceptable, c'est-à-dire compatible avec la peau et éventuellement avec ses phanères (cils, ongles, cheveux) et/ou les muqueuses. Ce milieu est avantageusement cosmétiquement acceptable, c'est-à-dire qu'il n'entraîne pas de démangeaisons, de picotements ou de rougeurs susceptibles de détourner l'utilisateur de la composition, et qu'il présente un aspect, une odeur et un toucher agréables.

Cette composition peut se présenter sous toutes les formes galéniques normalement utilisées dans le domaine cosmétique, et elle peut être notamment sous forme d'une solution éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les huiles, les cires, les émulsionnants, les gélifiants, les polymères filmogènes, les conservateurs, les parfums, les charges, les filtres UV, les bactéricides, les absorbeurs d'odeur, les matières colorantes, les actifs hydrophiles ou lipophiles, les extraits végétaux, les antioxydants. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des composés selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et co-émulsionnants utilisables dans l'invention, on peut citer par exemple les esters d'acide gras et de polyéthylène glycol tels que le stéarate de PEG-100, et les esters d'acide gras et de glycérine tels

que le stéarate de glycéryle.

Comme gélifiants / épaississants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants / épaississants lipophiles, on peut citer les argiles modifiées comme les bentones, les sels métalliques d'acides gras, et la silice hydrophobe.

Comme actifs, il sera avantageux d'introduire dans la composition utilisée selon l'invention au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les autres agents myorelaxants et/ou les agents dermo-décontractants ; les agents tenseurs ; les agents anti-pollution et/ou anti-radicalaire ; les agents agissant sur la microcirculation ; les agents agissant sur le métabolisme énergétique des cellules ; et leurs mélanges.

Des exemples de tels composés additionnels sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les protéines végétales et leurs hydrolysats, ainsi que les phytohormones ; les extraits marins tels que les extraits d'algues ; les extraits bactériens ; les sapogénines telles que la diosgénine et les extraits de Wild Yam en contenant ; les céramides ; les hydroxyacides ; les hydroxyacides, tels que l'acide salicylique et l'acide n-octanoyl-5-salicylique ; le resvératrol ; les oligopeptides et pseudodipeptides et leurs dérivés acylés ; les sels de manganèse et de magnésium, en particulier les gluconates ; et leurs mélanges.

Comme indiqué précédemment, la composition selon l'invention peut également renfermer des agents photo-protecteurs actifs dans l'UVA et/ou l'UVB, sous forme de composés organiques ou inorganiques, ces derniers étant éventuellement enrobés pour les rendre hydrophobes.

Les agents photo-protecteurs organiques peuvent notamment être choisis parmi : les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-3 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane.

Les agents photo-protecteurs inorganiques sont de préférence constitués d'oxyde de zinc et/ou de dioxyde de titane, de préférence de taille nanométrique, éventuellement enrobés d'alumine et/ou d'acide stéarique.

La composition selon l'invention est avantageusement destinée à être appliquée sur les zones du visage et/ou du front marquées par des rides d'expression, et/ou sur les personnes présentant des rides d'expression.

Les rides concernées sont de préférence celles disposées radialement autour de la bouche et/ou des yeux, en particulier les rides de la patte d'oie, et/ou situées au niveau du front, en particulier la ride dite du lion, située au niveau de la glabelle, dans l'espace inter-sourcillier, et/ou disposées horizontalement sur le front.

L'invention sera maintenant illustrée par les exemples non limitatifs suivants. Dans ces exemples, les quantités sont indiquées en pourcentage pondéral.

**Exemple 1** :

Synthèse du 1-(2-phényléthyl)-4-[N-(3-phénylpropyl)-amino pipéridine (composé 1)

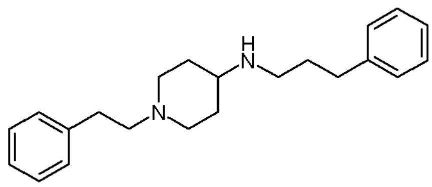

On a introduit 10 g de 1-phényléthyl-4-pipéridone (1 équivalent molaire) et 9,2 ml de 3-phénylpropylamine (1.3 équivalent molaire) dans 100 ml de dichlorométhane en présence de 3,09 ml d'acide acétique (1 équivalent molaire) et 21,94 g de $NaBH(OAc)_3$ (2 équivalent molaire), et on a laissé réagir à la température ambiante (25 °C) pendant 20 heures. Après traitement et purification, on a obtenu 15,8 g de produit sous forme d'huile marron (rendement 99 %).

Spectre de masse conforme à la structure attendue.

**Exemple 2** :

Synthèse du 1-(2-phényléthyl)-4-[N-éthyl-N-3-(phénylpropyl)]-amino pipéridine (composé 23)

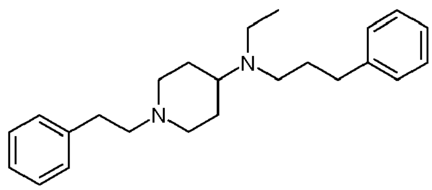

On a introduit 15,8 g de 1-(2-phényléthyl)-4-[N-(3-phénylpropyl)-amino pipéridine (obtenu selon l'exemple 1) et 3,65 ml de chlorure d'acétyle (1.05 équivalent molaire) dans le dichlorométhane en présence de 14,45 ml de triéthylamine (2.1 équivalent molaire) et on a laissé réagir à la température ambiante (25 °C) pendant 15 heures.
Après traitement et purification sur fritté de silice, on a obtenu 15,5 g de 1-(2-phényléthyl)-4-N-[acétyl-N-3-phénylpropyl)]-amino pipéridine (rendement 87 %).

Ensuite, 15,5 g du composé intermédiaire obtenu a été mélangé avec 8,5 g (5 équivalent molaire) de $LiAlH_4$ dans l'éther éthylique à reflux pendant 2 heures. Après traitement et purification sur fritté de silice, on a obtenu 10,5 g du produit attendu (Rendement 70 %).

**Exemple 3 :** Mise en évidence de l'effet dermo-décontractant des dérivés 4-amino-pipéridines selon l'invention

**a) Principe du test**

Le principe de ce test consiste à étudier l'effet du produit à tester sur un modèle de derme équivalent constitué d'une matrice de collagène ensemencée par des fibroblastes humains normaux.
Ces conditions sont destinées à mimer *in vitro* les phénomènes contractiles dermiques qui se produisent lors des mimiques du visage. Dans ces conditions, en effet, les cellules expriment spontanément des forces de traction qui induisent une rétraction du gel de collagène. Il en résulte une diminution de la surface totale du derme équivalent au cours du temps. La mesure de cette surface permet d'évaluer les effets de relaxation des substances préalablement mises en contact avec le derme équivalent.

**b) Protocole**

Deux séries de dermes équivalents attachés contenant des fibroblastes humains normaux sont préparés : une série témoin sans aucun traitement, et une série traitée par le composé à tester (1 μM). L'expérience est répétée trois fois.
Les équivalents de derme sont préparés comme décrits dans Asselineau et col., Exp. Cell. Res., 1985, 159, 536-539 ; Models in dermatology, 1987, vol 3 pp 1-7, dans les proportions suivantes :

| | |
|---|---|
| Milieu MEM (1,76X) avec ou sans composé | 45% |
| Sérum de Veau Foetal : | 10% |
| NaOH (0.1N) : | 5% |
| Acide acétique (1/1000) : | 4% |
| Collagène : | 26% |
| Fibroblastes : | 11% |

Le derme équivalent traité diffère du derme équivalent témoin en ce que l'on y ajoute 1 μM du composé à tester.
Le collagène utilisé est du collagène de type I (solution commerciale). Il est extrait de queues de rat ou de peau de veau par hydrolyse acide et conservé en milieu acide à +4°C ; il polymérise naturellement par réchauffement à 37°C et par diminution du taux d'acidité. Le collagène est préalablement dialysé contre des bains successifs d'eau + acide acétique.
Le protocole est le suivant : dans un tube à centrifuger de 50 ml conservé dans la glace pilée, on introduit le milieu MEM 1,76 X en présence d'additifs (Glutamine 1%, Acides aminés non essentiels 1%, Pyruvate de sodium 1%, Fungizone 1% et Pénicilline/Streptomycine 1%), le sérum de veau foetal, la soude NaOH 0,1 N. On ajoute alors les fibroblastes isolés à partir d'explants de peau humaine à la concentration de 1,5x $10^5$ cellules pour 1 ml de milieu de culture.
On ajoute alors lentement, contre la paroi du tube de façon à observer l'apparition d'un nuage blanchâtre, un mélange volume/volume de collagène dans de l'acide acétique au 1 /1000.
L'ensemble est alors mélangé avec précaution et réparti dans les puits d'une plaque de culture de 12 puits (type Costar référence 3512) à raison de 2ml de mélange par puits. La concentration cellulaire finale est de $3.10^4$ cellules/ derme équivalent, avec une concentration finale en collagène de 1 mg/ml. La plaque de culture est alors placée dans un incubateur à 37°C avec 5% de $CO_2$.
Une fois formés après polymérisation du collagène, les dermes équivalents sont laissés adhérents au support de culture pendant 3 jours puis détachés du support pour que la contraction puisse démarrer. Ces dermes équivalents attachés sont sortis de l'incubateur pour effectuer les prises d'image en vue de la mesure de leur surface et ce pour chaque point de la cinétique de contraction (0, 4, 8 et 24 heures). Ils sont immédiatement remis dans l'incubateur entre chaque point de mesure.
L'évaluation de la contraction spontanée des dermes équivalents traité (avec le composé à tester) et témoin (sans composé à tester) est réalisée en mesurant leur surface, à différents temps après le début de la contraction spontanée.
Pour cela, une image numérique est acquise pour chaque derme équivalent traité ou non traité au moyen d'une caméra (Caméra CCD -Iris Sony DXC -107P) et la surface est ensuite calculée sur chaque image au moyen d'un système d'analyse d'image (Zeiss Axiovision 3.0). A cette mesure de surface correspond un pourcentage de contraction égal au rapport des surfaces selon la formule :

$$\% \text{ contraction} = (Sp\text{-}Si)/Sp \times 100$$

où : 'Sp' représente la surface d'un puits de la plaque de culture ; elle correspond à la surface totale du derme équivalent avant contraction
'Si' représente la surface du derme équivalent à l'instant i de la cinétique de contraction.

**c) Résultats**

Le 1-(2-phényléthyl)-4-[N-(3-pénylpropyl)]-amino pipéridine (composé n° 1) de formule :

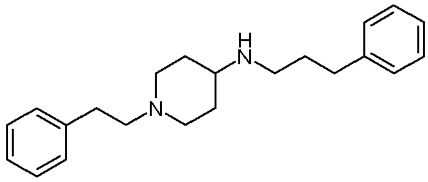

réduit la contraction des fibroblastes de 21 % en moyenne sur la durée de l'expérience (testé à 1 μM), par rapport au témoin.

Le 1-(2-phényléthyl) 4-[N-(1-pényléthyl)]-amino pipéridine (composé n°5) de formule :

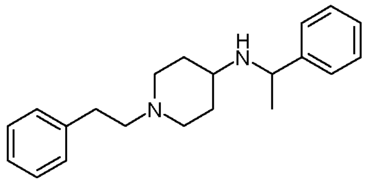

réduit la contraction des fibroblastes de 40 % en moyenne sur la durée de l'expérience (testé à 10 μM), par rapport au témoin.

Les 2 composés testés ont donc un effet dermo-décontractant significatif.

**Exemple 4 :**

On prépare une crème de soin de la peau ayant la composition suivante (pourcentage en poids) :

| | |
|---|---|
| Composé de l'Exemple 1 | 0,10 % |
| Acide stéarique | 3,00% |
| Mélange de mono-stéarate de glycéryle et stéarate de polyéthylène glycol (100 OE) | 2,50% |
| Stéarate de polyéthylène glycol (20 OE) | 1,00% |
| Cyclopentadiméthylsiloxane | 10,00% |
| Charges | 3,00% |
| Huiles végétales | 7,00% |
| Huiles synthétiques | 6,00% |
| Conservateurs | 1,20% |
| Diméthylsiloxane oxyéthyléné (16 OE) à extrémités méthoxy | 1,00% |
| Gomme de silicone | 0,20% |
| Copolymère acrylique en émulsion inverse (Simulgel 600 de SEPPIC) | 1,70% |
| Alcool stéarylique | 1,00% |
| Eau | qsp 100 % |

Cette crème est destinée à être appliquée sur le visage et le front pour détendre les traits et décontracter la peau du visage.

Dans la composition décrite précédemment, le composé n°1 peut être remplacé par le composé n°5 ou le composé n°23.

## Revendications

1. Utilisation cosmétique d'au moins un dérivé de 4-amino pipéridine de formule (I) :

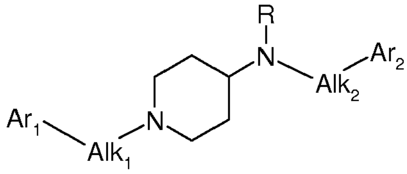

(I)

.

dans laquelle :

- $Alk_1$ et $Alk_2$ désignent, indépendamment l'un de l'autre, un radical alkylène linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, $-CF_3$, $-R_1$ , $-OR_1$ , $-NR_1R_2$ ;
- $Ar_2$ désigne un groupement phényle éventuellement substitué par un ou plusieurs radicaux, identiques ou différents, choisis parmi -F, $-CF_3$, $-NR_1R_2$ ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi $-Ar_1$, $-OR_1$, $-NR_1R_2$ ;

$R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ;
et leurs sels, isomères optiques et solvates,
comme agent pour lutter contre les rides, notamment les rides d'expression, et/ou décontracter la peau et/ou détendre les traits.

2. Utilisation selon la revendication précédente, **caractérisée par le fait que** le composé (I) est tel que :

- $Alk_1$ et $Alk_2$ désignent, indépendamment l'un de l'autre, un radical alkylène linéaire en $C_1$-$C_{10}$ ou ramifié en $C_3$-$C_{10}$ saturé ;
- $Ar_1$ désigne un groupement phényle éventuellement substitué par un ou plusieurs radicaux, identiques ou différents, choisis parmi $-R_1$ , $-OR_1$ ;
- $Ar_2$ désigne un groupement phényle éventuellement substitué par un ou plusieurs radicaux $-CF_3$ ;
- R désigne un atome d'hydrogène ou un radical alkyle linéaire en $C_1$-$C_4$, ou ramifié en $C_3$-$C_4$, saturé, éventuellement substitué par un groupement choisi parmi $-Ar_1$, $-OR_1$, - $NR_1R_2$ ;

$R_1$ et $R_2$ désignant, indépendamment l'un de l'autre, un radical alkyle saturé linéaire en $C_1$-$C_4$ .

3. Utilisation selon l'une quelconque des revendications précédentes, **caractérisée par le fait que** le composé (I) est tel que :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi un radical phényle, $-NR_1R_2$.

4. Utilisation selon l'une quelconque des revendications précédentes, **caractérisée par le fait que** le composé (I) est tel que :

- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;
- R désigne un atome d'hydrogène.

5. Utilisation selon la revendication 1, **caractérisée par le fait que** le composé (I) est choisi parmi :

- la 1-(2-phényléthyl)-4-[N-(3-phénylpropyl)]-amino pipéridine ;
- la 1-(2-phényléthyl)-4-(N-benzyl)-amino pipéridine ;
- la 1-(2-phényléthyl)-4-[N-(3,5-bistrifluorométhyl) benzyl]-amino pipéridine ;
- la 1-(2-phényléthyl)-4-[N-(2-phényléthyl)]-amino pipéridine ;
- la 1-(2-phényléthyl) 4-[N-(1-phényléthyl)]-amino pipéridine ;
- la 1-benzyl-4-[N-(1-phényléthyl)]-amino pipéridine ;
- la 1-[2-(3,4-diméthoxy)phényléthyl]- 4-[N-(3-phénylpropyl)]-amino pipéridine ;
- la 1-(3-phénylpropyl)-4-[N-(3-phénylpropyl)]-amino pipéridine ;
- la 1-(2-phényléthyl)-4-[N-(4-phénylbutyl)]-amino pipéridine ;
- la 1-(2-phényléthyl)-4-[N-(1-phénylpropyl)]-amino pipéridine ;
- la 1-[2-(4-tert-butyl)-phényléthyl]-4-[N-(3-phénylpropyl)]-amino pipéridine ;
- la 1-(2-phényléthyl)-4-[N-(1S)-1-phényléthyl]-amino pipéridine ;
- la 1-[2-(4-tert-butyl)-phényléthyl]-4-[N-(1-phényléthyl)]-amino pipéridine ;
- la 1-(4-phénylbutyl)-4-[N-(3-phénylpropyl)]-amino pipéridine ;
- la 1-(3-phénylpropyl)-4-[N-(1-phénylpropyl)]-amino pipéridine ;
- la 1-(3-phénylpropyl)-4-[N-(1S)-1-phényléthyl]-amino pipéridine ;
- la 1-(2-phényléthyl)-4-[N-méthyl-N-(3-phénylpropyl)]-amino pipéridine ;
- la 1-(2-phényléthyl)-4-[N,N-di-(2-phényléthyl)]-amino pipéridine ;
- la 1-(2-phényléthyl)-4-[N-éthyl-N-(1-phényléthyl)]-amino pipéridine ;
- la 1-(2-phényléthyl)-4-[N-(2-phénylpropyl)-N-(1-phényléthyl)]-amino pipéridine ;
- la 1-(2-phényléthyl)-4-[N-(3-phénylpropyl)-N-phényléthyl]amino pipéridine ;
- la 1-(2-phényléthyl)-4-[N-benzyl-N-(3-phénylpropyl)]-amino pipéridine ;
- la 1-(2-phényléthyl)-4-[N-éthyl-N-(3-phénylpropyl)]-amino pipéridine ;
- la 1-(2-phényléthyl)-4-[N-propyl-N-(3-phényl propyl)]-amino pipéridine ;
- la 1-(2-phényléthyl)-4-[N-butyl-N-3-(3-phényl propyl)]-amino pipéridine ;
- la 1-(2-phényléthyl)-4-[N-2-(3,4-diméthoxy)phényléthyl-N-(3-phénylpropyl)]-amino pipéridine ;
- la 1-(2-phényléthyl)-4-[N-2-diméthylaminoéthyl-N-(3-phénylpropyl)]-amino pipéridine ;
- la 1-(2-phényléthyl)-4-[N-2-méthoxyéthyl-N-(3-phénylpropyl)]-amino pipéridine.

**6.** Procédé de traitement cosmétique de la peau, notamment d'une peau ridée, en particulier la peau du visage et/ou du front, comprenant l'application topique sur ladite peau d'une composition comprenant, dans un milieu physiologiquement acceptable, au moins un dérivé de 4-amino pipéridine de formule (I) tel que défini selon l'une quelconque des revendications précédentes.

**7.** Composition comprenant, dans un milieu physiologiquement acceptable, au moins un dérivé de 4-amino pipéridine de formule (II) :

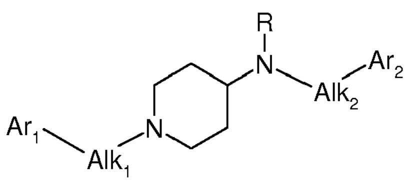

(II)

dans laquelle :

- $Alk_1$ désigne un radical alkylène linéaire en $C_2$-$C_{10}$ ou ramifié en $C_3$-$C_{10}$ , saturé ou insaturé ;
- $Alk_2$ désigne un radical alkylène linéaire saturé en $C_1$-$C_{10}$ ou insaturé en $C_2$-$C_{10}$ ou ramifié en $C_3$-$C_{10}$ saturé ou insaturé ;
- $Ar_1$ désigne un groupement phényle éventuellement substitué par un ou plusieurs radicaux, identiques ou différents, choisis parmi -F, -$CF_3$, -$R_1$, -$OR_1$, -$NR_1R_2$ ;
- $Ar_2$ désigne un groupement phényle éventuellement substitué par un ou plusieurs radicaux, identiques ou différents, choisis parmi -F, -$CF_3$, -$NR_1R_2$ ;
- R désigne un atome d'hydrogène ou un radical alkyle linéaire en $C_1$-$C_{10}$, ou ramifié en $C_3$-$C_{10}$, saturé ou insaturé, éventuellement substitué par un groupement choisi parmi -$Ar_1$, -$OR_1$, -$NR_1R_2$ ;

$R_1$ et $R_2$ désignant, indépendamment l'un de l'autre, un radical alkyle linéaire saturé en $C_1$-$C_7$, ou insaturé en $C_2$-$C_7$ ou ramifié ou cyclique en $C_3$-$C_7$ saturé ou insaturé ; et leurs sels, isomères optiques et solvates.

**8.** Composition selon la revendication précédente, **caractérisée par le fait que** le composé (II) est tel que :

- R désigne un atome d'hydrogène , et
- $Alk_1$ désigne un radical alkylène linéaire en $C_2$-$C_{10}$ ou ramifié en $C_3$-$C_{10}$ , saturé ; et
- $Alk_2$ désigne un radical alkylène linéaire en $C_1$-$C_{10}$ ou ramifié en $C_3$-$C_{10}$ , saturé ;

ou

- R désigne un radical alkyle linéaire en $C_1$-$C_4$, ou ramifié en $C_3$-$C_4$, saturé, éventuellement substitué par un groupement choisi parmi -$Ar_1$, -$OR_1$, -$NR_1R_2$ ; et
- $Alk_1$ et $Alk_2$ désignent, indépendamment l'un de l'autre, un radical alkylène linéaire en $C_1$-$C_{10}$ ou ramifié en $C_3$-$C_{10}$ saturé ;
- $Ar_1$ désigne un groupement phényle éventuellement substitué par un ou plusieurs radicaux, identiques ou différents, choisis parmi -$R_1$ , -$OR_1$ ;
- $Ar_2$ désigne un groupement phényle éventuellement substitué par un ou plusieurs radicaux -$CF_3$ ;

$R_1$ et $R_2$ désignant, indépendamment l'un de l'autre, un radical alkyle saturé linéaire en $C_1$-$C_4$ .

**9.** Composition selon l'une quelconque des revendications 7 et 8, **caractérisée par le fait que** le composé (II) est tel que :

- R désigne un atome d'hydrogène , et
- $Alk_1$ désigne un radical alkylène linéaire en $C_2$-$C_4$ ou ramifié en $C_3$-$C_4$, saturé ; et
- $Alk_2$ désigne un radical alkylène linéaire en $C_1$-$C_4$ ou ramifié en $C_3$-$C_4$ , saturé ;

ou

- R désigne un radical alkyle linéaire en $C_1$-$C_4$, ou ramifié en $C_3$-$C_4$, saturé, éventuellement substitué par un groupement choisi parmi -$Ar_1$, -$OR_1$, -$NR_1$ $R_2$ ; et
- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .

**10.** Composition selon l'une quelconque des revendications 7 à 9, **caractérisée par le fait que** le composé (II) est tel que :

- R désigne un atome d'hydrogène, et
- $Alk_1$ désigne un radical alkylène linéaire en $C_2$-$C_{10}$ ou ramifié en $C_3$-$C_{10}$ , saturé ; et
- $Alk_2$ désigne un radical alkylène linéaire en $C_1$-$C_{10}$ ou ramifié en $C_3$-$C_{10}$, saturé ;

ou

- R désigne un radical alkyle linéaire en $C_1$-$C_4$, ou ramifié en $C_3$-$C_4$, saturé, éventuellement substitué par un groupement choisi parmi un radical phényle, -$NR_1R_2$, et
- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$.

**11.** Composition selon l'une quelconque des revendications 7 à 10, **caractérisée par le fait que** le composé (II) est tel que :

- R désigne un atome d'hydrogène,

- $Alk_1$ désigne un radical alkylène linéaire en $C_2$-$C_{10}$ ou ramifié en $C_3$-$C_{10}$ , saturé ;
- $Alk_2$ désigne un radical alkylène linéaire en $C_1$-$C_{10}$ ou ramifié en $C_3$-$C_{10}$ , saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$.

**12.** Composition selon la revendication 7, **caractérisée par le fait que** le composé (II) est choisi parmi :

- la 1-(2-phényléthyl)-4-[N-(3-phénylpropyl)]-amino pipéridine ;
- la 1-(2-phényléthyl)-4-(N-benzyl)-amino pipéridine ;
- la 1-(2-phényléthyl)-4-[N-(3,5-bistrifluorométhyl) benzyl]-amino pipéridine ;
- la 1-(2-phényléthyl)-4-[N-(2-phényléthyl)]-amino pipéridine ;
- la 1-(2-phényléthyl) 4-[N-(1-phényléthyl)]-amino pipéridine ;
- la 1-[2-(3,4-diméthoxy)phényléthyl]- 4-[N-(3-phénylpropyl)]-amino pipéridine ;
- la 1-(3-phénylpropyl)-4-[N-(3-phénylpropyl)]-amino pipéridine ;
- la 1-(2-phényléthyl)-4-[N-(4-phénylbutyl)]-amino pipéridine ;
- la 1-(2-phényléthyl)-4-[N-(1-phénylpropyl)]-amino pipéridine ;
- la 1-[2-(4-tert-butyl)-phényléthyl]-4-[N-(3-phénylpropyl)]-amino pipéridine ;
- la 1-(2-phényléthyl)-4-[N-(1S)-1-phényléthyl]-amino pipéridine ;
- la 1-[2-(4-tert-butyl)-phényléthyl]-4-[N-(1-phényléthyl)]-amino pipéridine ;
- la 1-(4-phénylbutyl)-4-[N-(3-phénylpropyl)]-amino pipéridine ;
- la 1-(3-phénylpropyl)-4-[N-(1-phénylpropyl)]-amino pipéridine ;
- la 1-(3-phénylpropyl)-4-[N-(1S)-1-phényléthyl]-amino pipéridine ;
- la 1-(2-phényléthyl)-4-[N-méthyl-N-(3-phénylpropyl)]-amino pipéridine ;
- la 1-(2-phényléthyl)-4-[N,N-di-(2-phényléthyl)]-amino pipéridine ;
- la 1-(2-phényléthyl)-4-[N-éthyl-N-(1-phényléthyl)]-amino pipéridine ;
- la 1-(2-phényléthyl)-4-[N-(2-phénylpropyl)-N-(1-phényléthyl)]-amino pipéridine ;
- la 1-(2-phényléthyl)-4-[N-(3-phénylpropyl)-N-phényléthyl]amino pipéridine ;
- la 1-(2-phényléthyl)-4-[N-benzyl-N-(3-phénylpropyl)]-amino pipéridine ;
- la 1-(2-phényléthyl)-4-[N-éthyl-N-(3-phénylpropyl)]-amino pipéridine ;
- la 1-(2-phényléthyl)-4-[N-propyl-N-(3-phényl propyl)]-amino pipéridine ;
- la 1-(2-phényléthyl)-4-[N-butyl-N-3-(3-phényl propyl)]-amino pipéridine ;
- la 1-(2-phényléthyl)-4-[N-2-(3,4-diméthoxy)phényléthyl-N-(3-phénylpropyl)]-amino pipéridine ;
- la 1-(2-phényléthyl)-4-[N-2-diméthylaminoéthyl-N-(3-phénylpropyl)]-amino pipéridine ;
- la 1-(2-phényléthyl)-4-[N-2-méthoxyéthyl-N-(3-phénylpropyl)]-amino pipéridine.

**13.** Composition selon l'une quelconque des revendications 7 à 12, **caractérisée par le fait qu'**elle constitue une composition cosmétique ou dermatologique.

**14.** Composition selon l'une quelconque des revendications 7 à 13, **caractérisée par le fait qu'**elle comprend un ingrédient choisi parmi les huiles, les cires, les émulsionnants, les gélifiants, les polymères filmogènes, les conservateurs, les parfums, les charges, les filtres UV, les bactéricides, les absorbeurs d'odeur, les matières colorantes, les actifs hydrophiles ou lipophiles, les extraits végétaux, les antioxydants.

**15.** Composés de 4-amino-pipéridine de formule (III) :

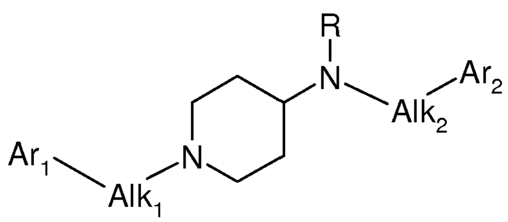

(III)

.

dans laquelle :

- $Alk_1$ désigne un radical alkylène linéaire en $C_2$-$C_{10}$ ou ramifié en $C_3$-$C_{10}$ , saturé ou insaturé ;
- $Alk_2$ désigne un radical alkylène linéaire ou ramifié, saturé ou insaturé, en $C_3$-$C_{10}$ ;
- $Ar_1$ désigne un groupement phényle éventuellement substitué par un ou plusieurs radicaux, identiques ou différents, choisis parmi -F, -$CF_3$, -$R_1$ , -$OR_1$ , -$NR_1$ $R_2$ ;
- $Ar_2$ désigne un groupement phényle éventuellement substitué par un ou plusieurs radicaux, identiques ou différents, choisis parmi -F, -$CF_3$, -$NR_1R_2$ ;
- R désigne un atome d'hydrogène ou un radical alkyle linéaire en $C_1$-$C_{10}$ ou ramifié en $C_3$-$C_{10}$, saturé ou insaturé, éventuellement substitué par un groupement choisi parmi -$Ar_1$, -$OR_1$, -$NR_1$ $R_2$ ;

$R_1$ et $R_2$ désignant, indépendamment l'un de l'autre, un radical alkyle, saturé ou insaturé, linéaire en $C_1$-$C_7$, ou ramifié ou cyclique en $C_3$-$C_7$ ;
et leurs sels, isomères optiques et solvates.

**16.** Composés selon la revendication précédente, **caractérisés par le fait que** :

- R désigne un atome d'hydrogène, et
- $Alk_1$ désigne un radical alkylène linéaire en $C_2$-$C_4$ ou ramifié en $C_3$-$C_4$ , saturé ; et
- $Alk_2$ désigne un radical alkylène linéaire ou ramifié en $C_3$-$C_4$ , saturé ;

ou

- R désigne un radical alkyle linéaire en $C_1$-$C_4$, ou ramifié en $C_3$-$C_4$, saturé, éventuellement substitué par un groupement choisi parmi -$Ar_1$, -$OR_1$, -$NR_1R_2$ ; et
- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .

**17.** Composés selon l'une quelconque des revendications 15 et 16, **caractérisés par le fait que** :

- $Alk_1$ désigne un radical alkylène linéaire en $C_2$-$C_4$ ou ramifié en $C_3$-$C_4$ , saturé ;
- $Alk_2$ désigne un radical alkylène linéaire ou ramifié en $C_3$-$C_4$ , saturé ;
- $Ar_1$ et $Ar_2$ désignent un groupement phényle ;
- R désigne un atome d'hydrogène ou un radical alkyle linéaire en $C_1$-$C_4$, ou ramifié en $C_3$-$C_4$, saturé, éventuellement substitué par un groupement choisi parmi -$Ar_1$, -$OR_1$, - $NR_1R_2$ ;

$R_1$ et $R_2$ désignant, indépendamment l'un de l'autre, un radical alkyle saturé linéaire en $C_1$-$C_4$ .

**18.** Composés selon l'une quelconque des revendications 15 à 17, **caractérisés par le fait que** :

- R désigne un atome d'hydrogène, et
- $Alk_1$ désigne un radical alkylène linéaire en $C_2$-$C_4$ ou ramifié en $C_3$-$C_4$ , saturé ; et
- $Alk_2$ désigne un radical alkylène linéaire ou ramifié en $C_3$-$C_4$ , saturé ;

ou

- R désigne un radical alkyle linéaire en $C_1$-$C_4$, ou ramifié en $C_3$-$C_4$, saturé, éventuellement substitué par un groupement choisi parmi un radical phényle, -$NR_1R_2$, et
- $Alk_1$ et $Alk_2$ désignent, indépendamment l'un de l'autre, un radical alkylène linéaire en $C_1$-$C_4$ ou ramifié en $C_3$-$C_4$ saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .

**19.** Composés selon l'une quelconque des revendications 15 à 18, **caractérisés par le fait que** :

- R désigne un atome d'hydrogène, et
- $Alk_1$ désigne un radical alkylène linéaire en $C_2$-$C_4$ ou ramifié en $C_3$-$C_4$ , saturé ; et
- $Alk_2$ désigne un radical alkylène linéaire ou ramifié en $C_3$-$C_4$ , saturé ;
- $Ar_1$ et $Ar_2$ désignent un groupement phényle ;

$R_1$ et $R_2$ désignant, indépendamment l'un de l'autre, un radical alkyle saturé linéaire en $C_1$-$C_4$ .

**20.** Composés selon la revendication 15, **caractérisés par le fait qu'**ils sont choisis parmi les composés suivants :

- la 1-(2-phényléthyl)-4-[N-(3-phénylpropyl)]-amino pipéridine ;
- la 1-[2-(3,4-diméthoxy)phényléthyl]- 4-[N-(3-phénylpropyl)]-amino pipéridine ;
- la 1-(3-phénylpropyl)-4-[N-(3-phénylpropyl)]-amino pipéridine ;
- la 1-(2-phényléthyl)-4-[N-(4-phénylbutyl)]-amino pipéridine ;
- la 1-(2-phényléthyl)-4-[N-(1-phénylpropyl)]-amino pipéridine ;
- la 1-[2-(4-tert-butyl)-phényléthyl]-4-[N-(3-phénylpropyl)]-amino pipéridine ;
- la 1-(4-phénylbutyl)-4-[N-(3-phénylpropyl)]-amino pipéridine ;
- la 1-(3-phénylpropyl)-4-[N-(1-phénylpropyl)]-amino pipéridine ;
- la 1-(3-phénylpropyl)-4-[N-(1S)-1-phényléthyl]-amino pipéridine ;
- la 1-(2-phényléthyl)-4-[N-méthyl-N-(3-phénylpropyl)]-amino pipéridine ;
- la 1-(2-phényléthyl)-4-[N,N-di-(2-phényléthyl)]-amino pipéridine ;
- la 1-(2-phényléthyl)-4-[N-éthyl-N-(1-phényléthyl)]-amino pipéridine ;
- la 1-(2-phényléthyl)-4-[N-(2-phénylpropyl)-N-(1-phényléthyl)]-amino pipéridine ;
- la 1-(2-phényléthyl)-4-[N-(3-phénylpropyl)-N-phényléthyl]amino pipéridine ;
- la 1-(2-phényléthyl)-4-[N-benzyl-N-(3-phénylpropyl)]-amino pipéridine ;
- la 1-(2-phényléthyl)-4-[N-éthyl-N-(3-phénylpropyl)]-amino pipéridine ;
- la 1-(2-phényléthyl)-4-[N-propyl-N-(3-phényl propyl)]-amino pipéridine ;
- la 1-(2-phényléthyl)-4-[N-butyl-N-3-(3-phényl propyl)]-amino pipéridine ;
- la 1-(2-phényléthyl)-4-[N-2-(3,4-diméthoxy)phényléthyl-N-(3-phénylpropyl)]-amino pipéridine ;
- la 1-(2-phényléthyl)-4-[N-2-diméthylaminoéthyl-N-(3-phénylpropyl)]-amino pipéridine ;
- la 1-(2-phényléthyl)-4-[N-2-méthoxyéthyl-N-(3-phénylpropyl)]-amino pipéridine.

**21.** Procédé de préparation d'un composé selon l'une quelconque des revendications 15 à 19 et pour lequel R désigne un atome d'hydrogène, comprenant l'alkylation du iodure du 1,1-diméthyl 1,1-diméthyl-4-oxopipéridinium avec une alkylamine (III) pour former une pipéridone (A) , la pipéridone (A) étant ensuite alkylée et réduite avec une alkylamine (B) pour conduire au composé (Ia) :

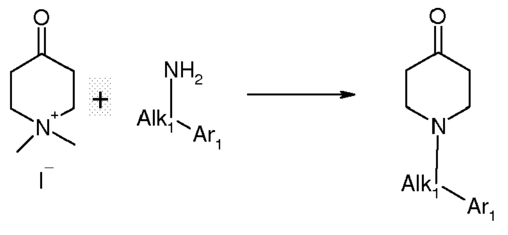

(A)

Schéma I

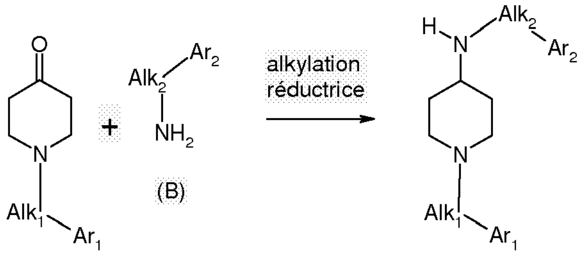


(A) (B) (Ia)

Schéma II

**22.** Procédé de préparation d'un composé selon l'une quelconque des revendications 15 à 19 et pour lequel R est différent d'un atome d'hydrogène, comprenant les étapes suivantes :

le composé (Ia) est acylé par le chlorure d'acide de formule RCOCl en présence d'un base organique pour conduire à un amide intermédiaire qui est réduit en présence de $LiAlH_4$ et conduit au composé (Ib) :

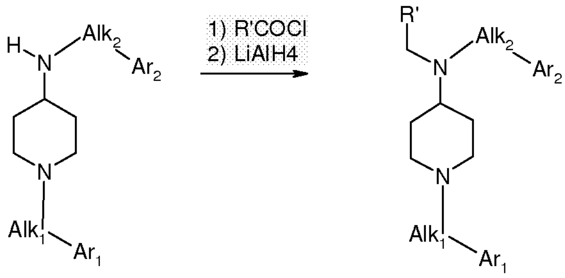


(Ia) (Ib)

Office européen des brevets

# RAPPORT DE RECHERCHE EUROPEENNE

Numéro de la demande
EP 07 10 5821

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 3 531 487 A (BERGER LEO ET AL) 29 septembre 1970 (1970-09-29)<br>* exemple 16 *<br>----- | 15,16 | INV.<br>C07D211/58<br>A61K8/49 |
| X | JANSSENS F ET AL: "NEW ANTIHISTAMINIC N-HETEROCYCLIC 4-PIPERIDINAMINES. 1. SYNTHESIS AND ANTIHISTAMINIC ACTIVITY OF N-(4-PIPERIDINYL)-1H-BENZIMIDAZOL-2-AMINES "<br>JOURNAL OF MEDICINAL CHEMISTRY, AMERICAN CHEMICAL SOCIETY. WASHINGTON, US,<br>vol. 28, no. 12, 1985, pages 1925-1933, XP002074741<br>ISSN: 0022-2623<br>composé 95<br>----- | 15 | |
| X | CASY A F ET AL: "STRUCTURE-ACTIVITY STUDIES OF FENTANYL"<br>JOURNAL OF PHARMACY AND PHARMACOLOGY, LONDON, GB,<br>vol. 40, 1988, pages 605-608, XP001037232<br>ISSN: 0022-3573<br>préparation de 1-phénéhtyl-piperidin-4-(N-phénéthylamine) , (page 606, préparation de composé 2b)<br>----- | 15 | DOMAINES TECHNIQUES RECHERCHES (IPC)<br>C07D<br>A61K |
| X | WALKER G N ET AL: "APPLICATION OF SODIUM BOROHYDRIDE, REDUCTION TO SYNTHESIS OF SUBSTITUTED AMINOPIPERIDINES, AMINOPIPERAZINES, AMINOPYRIDINES AND HYDRAZINES"<br>JOURNAL OF ORGANIC CHEMISTRY, AMERICAN CHEMICAL SOCIETY. EASTON, US,<br>vol. 26, no. 8, 14 août 1961 (1961-08-14), pages 2740-2747, XP002071843<br>ISSN: 0022-3263<br>* tableau III *<br>-----<br>-/-- | 15 | |

Le présent rapport a été établi pour toutes les revendications

| Lieu de la recherche | Date d'achèvement de la recherche | Examinateur |
|---|---|---|
| La Haye | 26 juillet 2007 | Diederen, Jeroen |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

Office européen des brevets

# RAPPORT DE RECHERCHE EUROPEENNE

Numéro de la demande
EP 07 10 5821

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | HARPER N J ET AL: "THE CHEMISTRY AND PHARMACOLOGY OF SOME 4-AMINOPIPERIDINES AND THEIRDERIVATIVES" JOURNAL OF MEDICINAL CHEMISTRY, AMERICAN CHEMICAL SOCIETY. WASHINGTON, US, vol. 7, novembre 1964 (1964-11), pages 729-732, XP001037233 ISSN: 0022-2623 1-benzyl-4-benzylaminopipéridine ----- | 15 | |
| A | FR 2 847 345 A1 (OREAL [FR]) 21 mai 2004 (2004-05-21) * exemple 1 * ----- | 1,6,7, 15,21,22 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| La Haye | 26 juillet 2007 | Diederen, Jeroen |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 07 10 5821

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-07-2007

| Document brevet cité au rapport de recherche | | Date de publication | Membre(s) de la famille de brevet(s) | Date de publication |
|---|---|---|---|---|
| US 3531487 | A | 29-09-1970 | AUCUN | |
| FR 2847345 | A1 | 21-05-2004 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005005395 A
- HU 157777325
- WO 02083641 A


### Littérature non-brevet citée dans la description


- **J.D. CARRUTERS et al.** *J. Dermatol. Surg. Oncol.,* 1992, vol. 18, 17-21
- **CORRUBLE A. et al.** Structure-selectivity relationship in alkyllithium-aldehyde condensations using 3-aminopyrrolidine lithium amides as chiral auxiliaries. *J. Org. Chem,* 1998, vol. 63, 8266-8275
- **ASSELINEAU.** *Exp. Cell. Res.,* 1985, vol. 159, 536-539
- *Models in dermatology,* 1987, vol. 3, 1-7